Europäisches Patentamt

European Patent Office

Office européen des brevets

(11) Veröffentlichungsnummer: **0 195 213 B1**

(12) **EUROPÄISCHE PATENTSCHRIFT**

(45) Veröffentlichungstag der Patentschrift: **28.04.93**

(21) Anmeldenummer: **86101444.7**

(22) Anmeldetag: **04.02.86**

Ein Antrag gemäss Regel 88 EPÜ auf Berichtigung der Beschreibung liegt vor. Über diesen Antrag wird im Laufe des Verfahrens von der Prüfungsabteilung eine Entscheidung getroffen werden.

(51) Int. Cl.5: **A23L 2/02**, A23L 2/34, A23L 1/064, A23L 1/068, A23B 7/10

(54) **Milchsäurehaltige Fruchtprodukte und Verfahren zur Lactofermentation von Fruchtprodukten.**

(30) Priorität: **05.02.85 DE 3503742**

(43) Veröffentlichungstag der Anmeldung:
**24.09.86 Patentblatt 86/39**

(45) Bekanntmachung des Hinweises auf die Patenterteilung:
**28.04.93 Patentblatt 93/17**

(84) Benannte Vertragsstaaten:
**AT BE CH DE FR LI LU NL**

(56) Entgegenhaltungen:
EP-A- 0 113 055       DE-A- 1 926 166
DE-A- 2 440 516       FR-A- 383 707
FR-A- 1 178 657       FR-A- 2 075 591
US-A- 1 417 412       US-A- 3 403 032

(73) Patentinhaber: **ECKES AG**
**Ludwig-Eckes-Allee 8**
**W-6501 Nieder-Olm(DE)**

(72) Erfinder: **Wiesenberger, Alfred**
**Haideweg 29**
**W-6200 Wiesbaden-Sonnenberg(DE)**
Erfinder: **Kolb, Erich, Dr. Dipl.-Ing.**
**Wilhelm-Holzamer-Weg 68**
**W-6501 Nieder-Olm(DE)**
Erfinder: **Schildmann, Jens A., Dipl.-Ing.**
**Heerstrasse 38**
**W-6501 Partenheim(DE)**
Erfinder: **Dechent, Hans-Mario**
**In der Siedlung 1**
**W-6501 Saulheim(DE)**

(74) Vertreter: **Fuchs, Jürgen H., Dr.-Ing. et al**
**Dr. Fuchs, Dr. Luderschmidt Dr. Mehler,**
**Dipl.-Ing. Weiss Patentanwälte Abraham-**
**Lincoln-Strasse 7 Postfach 46 60**
**W-6200 Wiesbaden (DE)**

**Beschreibung**

Die Erfindung betrifft ein Verfahren zur Lactofermentation von Fruchtprodukten, bei dem das Ausgangsproduxt in Form von Maische oder Saft nach Abtöten der in ihm enthaltenen wilden Mikroflora einer Fermentation durch milchsäureerzeugende Bakterien unterzogen wird, die als Fermentationsprodukt mindestens 95 % L(+)-Milchsäure erzeugen.

Das erfindungsgemäße Verfahren kann vorteilhafterweise an aus Früchten hergestellten Säften oder anderen aus Früchten hergestellten Ausgangsprodukten angewandt werden, die einen pH-Wert von weniger als 3.7 aufweisen.

Zu derartigen Früchten gehören sämtliche Früchte, die einen relativ sauren Geschmack aufweisen, wie beispielsweise Äpfel, Weiße und rote Weintrauben, Aprikosen, Sauerkirschen, Pfirsiche, Birnen, Orangen, schwarze und rote Johannisbeeren und dergleichen.

Mit dem erfindungsgemäßen Verfahren werden aus diesen Früchten hergestellte Ausgangsprodukte, beispielweise Säfte, Nektar, Mark oder Pulpe oder Mischungen davon behandelt. Als Saft einsetzbar sind klare und trübe Säfte, die durch Abpressen aus diesen Früchten hergestellt worden sind.

Unter Nektar, der sowohl in einer deutschen als auch in einer EG-Verordnung definiert ist, ist ein Fruchtnektar oder Fruchtsyrup zu verstehen, d.h. das nicht gegorene, aber gärfähige, durch Zusatz von Wasser und/oder Zucker zu gegebenenfalls konzentriertem Fruchtsaft oder gegebenenfalls konzentriertem Fruchtmark oder einem Gemisch derselben hergestellte Erzeugnis, wobei dieses hergestellte Erzeugnis entsprechend den nachstehenden Angaben durch einen Mindestfruchtgehalt und einen Mindestsäuregehalt definiert ist. So soll ein Nektar aus schwarzen oder roten Johannisbeeren einen Fruchtgehalt von mindestens 25 Gew.-% und einen Säuregehalt von mindestens 8 Promille (gerechnet als Weinsäure), also 8 g/l Nektar enthalten. In ähnlicher Weise soll ein Sauerkirschnektar mindestens 35 Gew.-% Fruchtgehalt und mindestens 8 Promille Säure, Aprikosennektar mindestens 40 Gew.-% Fruchtgehalt und mindestens 6 Promille Säure und ein Pfirsichnektar mindestens 45 Gew.-% Fruchtgehalt und mindestens 3 Promille Säure enthalten.

Der Einsatz des erfindungsgemäßen Verfahrens ist jedoch nicht auf derartige Nektare beschränkt. Demzufolge kann das erfindungsgemäße Verfahren auch an Nektaren durchgeführt werden, die andere, d.h. höhere und tiefere Frucht- oder Säuregehalte aufweisen.

Wie bereits vorstehend erwähnt, kann das erfindungsgemäße Verfahren auch an einem Fruchtmark durchgeführt werden. Unter Mark ist der eßbare Teil der geschälten und entkernten Frucht zu verstehen, der durch passieren oder ein ähnliches Verfahren zu Mark zerkleinert worden ist.

Milchsäureerzeugende Fermentationen sind seit langem bekannt. Beispielhaft sei nur die Herstellung von Sauerkraut erwähnt. Die Milchsäurefermentation wurde in früheren Zeiten vornehmlich dazu eingesetzt, um bestimmte feuchte Lebensmittel haltbarer zu machen. Später wurde mit der Milchsäure-Vergärung auch das Ziel verfolgt, vorwiegend Gemüse-Maischen und -Säfte durch einen besseren Aufschluß des Nahrungsmittels und durch die gebildete Säure gehaltlich und sensorisch aufzuwerten. Bei der herkömmlichen heterofermentativen Milchsäure-Fermentation entstehen in vielen Fällen zusätzlich aber auch unerwünschte Nebenprodukte, die den durch die Vergärung angestrebten sensorischen Gewinn in Frage stellen.

In jüngster Zeit begann man darauf Wert zu legen, daß bei der Milchsäurefermentation nicht nur eine Säuerung des Produktes durch die Erzeugung von Milchsäure schlechthin erzeugt wird, ohne Beachtung, ob diese links- oder rechtsdrehend ist, sondern daß vorwiegend die physiologisch wirksame L(+)-Milchsäure gebildet wird. Zu diesem Zweck sind spezielle homofermentativ arbeitende Bakterienkulturen ausgewählt worden, die in der Lage sind, überwiegend L(+)-Milchsäure zu erzeugen.

Die Anwesenheit von L(+)-Milchsäure in fast allen Organen und Geweben des menschlichen Organismus beweist deren Bedeutung für die Zellreaktionen. Je größer die energetische Organleistung ist, um so höher steigt der Bedarf an L(+)-Milchsäure. Ein ausgewogener Stoffwechsel erfordert daher immer ein Potential an L(÷)-Milchsäure, das die Funktionen der Organe und Gewebe, insbesondere von Muskeln, Leber und Herz aufrechterhält. Das Angebot an L(+)-Milchsäure in der üblichen täglichen Nahrung liegt in der Regel unter dem Bedarf des Stoffwechsels an dieser Substanz, weswegen es wünschenswert ist, insbesondere solche Nahrungsmittel mit L(+)-Milchsäure anzureichern, aus denen die L(+)-Milchsäure sofort stoffwechselverfügbar ist.

Ein in diese Richtung weisendes Verfahren ist in der DE-PS 2 001 874 beschrieben, welches die Herstellung von milchsauren Gemüse- oder Fruchtsäften zum Gegenstand hat. In der zitierten Patentschrift werden vier spezielle Bakterienstämme beschrieben, darunter auch Lactobacillus casei, mit denen Gemüse- oder Fruchtmaischen bzw. -säfte nach Abtöten der in ihnen enthaltenen wilden, milchsäureerzeugenden Bakterien einer Fermentation unterzogen werden sollen, bei der Milchsäure gebildet wird, die zumindestens 90 % aus L(+)-Milchsäure besteht. Als Beispiele werden in dieser Veröffentlichung die Vergärung von Karottenmaische, von Karottensaft, von Saft Roter Rüben und von Bananenmaische beschrieben. Dabei handelt es sich durchweg um Produkte, die einen sehr geringen Ausgangssäuregehalt aufweisen, d.h. einen pH-Wert zwischen etwa 5,0 und 6,0. Die Gärung wird gezielt zur Säuerung der Produkte durchgeführt und erreicht gemäß den Angaben in der Patentschrift nach einer Fermentationszeit von 12 bis 20 Stunden einen End-pH-Wert von etwa 3,7 bis 3,9. Nach den sich an die Ausführungsbeispiele anschließenden Tabellen werden mit bestimmten dort angegebenen Mischungen von Bakterienkulturen nach einer Fermentationszeit von 24 Stunden zwar auch noch etwas niedrigere pH-Werte erreicht, jedoch lassen diese Ausführungsbeispiele erkennen, daß mit zunehmender Fermentationszeit und Erreichen geringerer pH-Werte sich das Verhältnis zwischen L(+)-Milchsäure und D(-)-Milchsäure stark verschiebt, so daß in einem Fall maximal noch ein Gehalt an L(+)-Milchsäure von 90 %, in den übrigen Fällen aber nur noch ein Gehalt an L(+)-Milchsäure von 50 bis 60 % erzielt wird.

Ein weiteres Merkmal des vorbekannten Verfahrens besteht darin, daß die Vermehrung der Mikroorganismen im zu fermentierenden Produkt selbst erfolgt. Es wird zwar in kleineren Mengen des gleichen Produktes vorher eine Anreicherung von Mikroorganismen erzeugt, mit denen die Hauptmenge dann beimpft wird, diese Beimpfungsmenge ist jedoch im wesentlichen nur so groß, daß dadurch eine weitere Vermehrung der Mikroorganismen in der Hauptmenge ausgelöst und die Fermentation somit unter starker Vermehrung der Mikroorganismen durchgeführt wird. Hierbei können während der Vermehrung resultierende Nebenprodukte, die einem einwandfreien sensorischen Ergebnis u.U. abträglich sind, anfallen.

In der DE-PS 24 40 516 wird ein weiterer Bakterienstamm namens Lactobacillus bavaricus zur milchsauren Vergärung von pflanzlichem Material beschrieben. Die in dieser Patentschrift wiedergegebenen Ausführungsbeispiele betreffen die Herstellung von Sauerkraut und Salzgurken. Der pH-Wert nach 20 Tagen liegt bei 3,9 bzw. 3,8.

Der Erfindung liegt die Aufgabe zugrunde, ein Verfahren zur Lactofermentation von Fruchtprodukten aufzuzeigen, mit dem es möglich ist, in Fruchtmaterial, dessen Ausgangsazidität schon verhältnismäßig hoch liegt, dennoch durch Fermentation nennenswerte Mengen von fast ausschließlich der physiologisch wirksamen L(+)-Milchsäure zu erzeugen, ohne daß der sensorische Charakter der Produkte nachteilig beeinflußt, sondern möglichst noch verbessert wird. Es kommt bei dem angestrebten Verfahren nicht darauf an, aus sensorischen Gründen eine gewisse Säuerung eines wenig sauren Ausgangsproduktes zu erreichen, wie dies bei herkömmlichen Verfahren der Fall ist, sondern in den von Natur aus bereits verhältnismäßig sauren Fruchtausgangsmaterialien einen Teil der darin enthaltenen Substanzen in L(+)-Milchsäure umzuwandeln, ohne daß sich die Bakterien unter zusätzlichem Abbau von Zuckeranteilen des Saftes wesentlich vermehren.

Es wurde überraschenderweise gefunden, daß auch die eingangs genannten Produkte mit einem Ausgangs-pH-Wert von 3,7 oder vorzugsweise von 3,2 - 3,6 mit milchsäureerzeugenden Bakterien behandelt werden können. Dabei wird im weit überwiegenden Maß die in den Fruchtprodukten vorhandene Äpfelsäure in Milchsäure umgewandelt.

Die vorstehend genannte Aufgabe wird daher erfindungsgemäß dadurch gelöst, daß Bakterien verwendet werden, deren milchsäureerzeugende Stoffwechselvorgänge auch noch bei pH-Werten kleiner 3,7 in Gang gebracht werden und ablaufen, und daß die Fermentation bei PH-Werten kleiner 3,7 durchgeführt und dabei ein Anteil an L(+)-Milchsäure von mindestens 95 Gew.-% gebildet wird. Vorzugsweise werden Ausgangsprodukte mit pH-Werten von 3,6 bis 3,2 verarbeitet.

Das Verfahren kann so geführt werden, daß der PH-Wert am Ende der Fermentation kleiner als 3,5 ist.

Es hat sich gezeigt, daß bei der milchsauren Fermentation bei pH-Werten kleiner 3,7 nicht nur gewisse Zucker zu Milchsäure vergoren werden, sondern daß von den benutzten Bakterienstämmen auch ein Teil der in den Ausgangsprodukten enthaltenen organischen Säuren, insbesondere der Äpfelsäure, in Milchsäure umgewandelt wird. Hierdurch wird z.T. ein sensorischer Gewinn erzielt, da insbesondere die geschmacklich auch stärker saure Äpfelsäure durch die sich angenehmer auswirkende, mildere Milchsäure ersetzt wird. Demzufolge weist das Fermentationsprodukt im allgemeinen keinen mit Sicherheit nachweisbaren Äpfelsäuregehalt mehr auf, vorzugsweise enthält es einen Äpfelsäuregehalt von nicht mehr als 0,1 Gew.-% bzw. von nicht mehr als 0,2 bzw. 0.5 Gew.-%. Bei nicht verdünnten Ausgangsprodukten kann u.U. ein Äpfelsäuregehalt von maximal 1.0 Gew.-% gegeben sein. Ein entsprechend höherer Wert kann sich natürlich bei Weiterverarbeitung des Fermentationsproduktes zu einem Endprodukt ergeben, wenn das Fermentationsprodukt konzentriert oder mit unvergorenem Ausgangsprodukt vermischt wird.

Das Fermentationsprodukt kann zu verkaufsfertigen Fruchtsäften, Konfitüren, Gelees, Brotaufstrichen und Fruchtriegeln aufbereitet werden.

Für unverdünnte Fruchtmasse (Saft oder Mark) gelten als Anhaltswerte folgende Gehalte an Äpfelsäure: Orangen 1-2 g/l, Äpfel 4-5 g/l, Trauben 4-5 g/l, Pfirsiche über 2 g/l, Aprikosen 4-5 g/l und Sauerkirschsaft etwa 17 g/l. Bei diesen Ausgangsprodukten tritt eine weitgehende Beseitigung des Äpfelsäuregehaltes ein.

EP 0 195 213 B1

Als Ausnahme weisen Saft und Nektar aus schwarzen Johannisbeeren einen sehr geringen Gehalt an Äpfelsäure auf.

Auch ist beim erfindungsgemäßen Verfahren eine Aufsäuerung mancher Citronensäure enthaltender Produkte, wie z.B. Pfirsichpulpe erwünscht. Hierbei wird die L(+)-Milchsäure aus Zucker gebildet.

Das Verfahren wird so geführt, daß bei der Fermentation mindestens 5 g L(+)-Milchsäure pro Liter Fermentationsprodukt, vorzugsweise mindestens 6 g/l erzeugt werden. Je nach Rohstoff und Verfahrensbedingungen ist es jedoch auch möglich, höhere Milchsäuregehalte zu erreichen. Insofern solche höheren Milchsäuregehalte nicht erforderlich sind, kann das Fermentationsprodukt nachträglich mit unvergorenem Produkt auf einen Gehalt von 5 g je Liter eingestellt werden.

Beim beschriebenen Verfahren bleibt der pH-Wert des erhaltenen Produktes regelmäßig unter einem Wert von 3,7, vorzugsweise in einem Bereich von 3,0 bis 3,6, insbesondere von 3,1 bis 3,4 vorzugsweise 3,5. Durch die Umwandlung der Äpfelsäure, die schwächer sauer ist als die Milchsäure, weist das erhaltene Produkt regelmäßig einen etwas höheren Säuregehalt als das Ausgangsprodukt auf, d.h. der pH-Wert des erhaltenen Produktes ist üblicherweise etwas geringer als der pH-Wert des Ausgangsproduktes. Da die Natursäfte in aller Regel eine hohe Pufferkapazität aufweisen, kann jedoch aber der End-pH-Wert dem Ausgangs-pH-Wert entsprechen. Während der Fermentation kann der pH-Wert sogar um etwa 0,1 ansteigen, wobei jedoch festzustellen ist, daß erfindungsgemäß der Ausgangs-pH-Wert regelmäßig um 0,1 bis 0,2 Einheiten höher ist als der End-pH-Wert, wie dies vorstehend festgestellt worden ist.

Eine besonders zweckmäßige Ausgestaltung des Verfahrens besteht darin, daß die Bakterien, d.h. die Biomasse, dem zu fermentierenden Ausgangsprodukt als Reinzuchtkonzentrat zugesetzt werden. Das Schneeballsystem der Keimanreicherung im zu fermentierenden Produkt ist wegen seiner sensorisch negativen Folgeerscheinungen insbesondere in Fruchtsafterzeugnissen unerwünscht. Deshalb werden erfindungsgemäß für die Fermentation entsprechende Mengen der ausgewählten Mikroorganismen zugegeben, um die angestrebte Menge an L(+)-Milchsäure als Stoffwechselprodukt der Organismen zu erhalten.

Bei der erfindungsgemäß angestrebten Verfahrensführung findet insgesamt keine Vermehrung der Mikroorganismen statt. Die Absterberate liegt vielmehr im Bereich von 50 %. Nach Beendigung der Fermentation sind noch etwa 1/3 bis 2/3 der ursprünglich zugegebenen Menge an lebender Biomasse im Produkt vorhanden.

Bei bevorzugter Ausgestaltung des beschriebenen Verfahrens wird die verbleibende Biomasse am Ende aus dem Produkt entfernt, da die erhaltenen Fermentationsprodukte eigentliches Ziel des Verfahrens sind. Eine solche Entfernung der Biomasse bietet sich im wesentlichen bei der Herstellung von klaren Fruchtsaftprodukten an. Andererseits ist es dem Verfahrensergebnis aber nicht abträglich, wenn, bedingt durch die Art des Produktes, die nach der Fermentation verbleibende Restmenge an Biomasse im Produkt belassen wird.

Die Anzucht der Bakterienstämme erfolgt in einem getrennten, an sich bekannten Fermentationsverfahren unter optimalen Ernährungs- und Vermehrungsbedingungen, wie dies weiter unten noch näher beschrieben wird.

Dem zu fermentierenden Ausgangsprodukt sollen dementsprechend mindestens 1 g, in der Regel aber 2 bis 10 g feuchte gereinigte Biomasse je Liter Ausgangsprodukt zugegeben werden. Aus ökonomischen Gründen wird sich die Zugabemenge im wesentlichen zwischen 2 und 6 g bewegen. Die hier angegebenen Mengen beziehen sich auf eine gereinigte Bakterienmasse mit einem Trockengehalt von 21-25 %, im allgemeinen von etwa 23 %. Die Keimzahl beträgt etwa $10^{12}$ je Gramm dieser Biomasse.

Die Fermentationszeiten beim beschriebenen Verfahren liegen im allgemeinen bei mindestens 24 Stunden, können aber in Sonderfällen auch bis 96 bzw. 100 Stunden ausgedehnt werden. Die zweckmäßigste Fermentationszeit ergibt sich aus wirtschaftlichen Erwägungen in Zusammenhang mit der zugesetzten Biomasse und der gewählten Fermentationstemperaturen. Diese sind zwischen 15 und 40°C möglich, Temperaturen zwischen 25°C und 35°C werden bevorzugt angewandt. Bei besonders empfindlichen Produkten kann es jedoch erstrebenswert sein, die Fermentation auch unterhalb von 20°C ablaufen zu lassen, weswegen die verwendeten Bakterienstämme auch hierfür geeignet sind.

Für die Durchführung des beschriebenen Verfahrens wurden einige geeignete Bakterienstämme ausgewählt bzw. isoliert. Ein in diesem Zusammenhang isolierter und für die Durchführung des Verfahrens geeigneter Mikroorganismus von der Gattung Lactobacillus war mit der Bezeichnung Lacobacillus spec. zu versehen. In Versuchen wurde dieser Stamm mit Beta 8 bezeichnet. Kulturen dieses Organismus sind unter der Nr. DSM 3174 bei der Deutschen Sammlung für Mikroorganismen in Göttingen hinterlegt worden. Dieser Mikroorganismus wurde aus Orangensaft selektiert, dann zunächst auf Rogosa Agar angezüchtet und in MRS-Broth (vertrieben von der Firma OXOID unter der Artikel-Nr. CM 359) überführt.

Es handelt sich dabei um bewegliche Lactobacillen, die meso-Diaminopimelinsäure in ihrer Zellwand enthalten. Von den bekannten Lactobacillusarten, auf die diese Merkmale zutreffen (L. agilis, L. yamanas-

4

EP 0 195 213 B1

hiensis und L. ruminis), unterscheiden sich die Stämme in einer Reihe von Merkmalen, wie Wachstumstemperatur, Glukonatverwertung und Basenzusammensetzung der DNS, so daß es sich um einen Vertreter bisher nicht beschriebener Art handelt.

Lactobacillus spec. besteht aus grampositiven Stäbchen und bildet keine Sporen. Katalase- und Nitratreduktase sind negativ. Es ist ein fakultativ anaerober Stamm. Die Mikroorganismen sind homofermentativ, Glukose wird ausschließlich zu Milchsäure vergoren. Die gebildete Milchsäure besteht zu 95 bis 99 % aus L( + )-Anteil.

Zu Milchsäure vergoren werden durch diesen Lactobacillus Ribose, Mannit, Sorbit, Maltose, Saccharose, Cellobiose, Trehalose, Salicin und Glucose. Keine Milchsäure gebildet wird aus Arabinose, Xylose, Rhamnose, Lactose, Melibiose, Raffinose und Melezitose. Das Wachstum des neuen Bacillus bei 15°C ist positiv, bei 45°C negativ. Gasentwicklung aus Gluconat positiv.

Die Anzucht von Lactobacillus spec. für die Durchführung des vorliegenden Verfahrens erfolgt in MRS-Broth. 1 g Biomasse pro Liter Broth bildet nach 24 Stunden bei 33°C die 15-fache Menge Biomasse. In einem im Handel erhältlichen Laborfermenter wird eine BETA-Nährlösung steril vorgelegt, durch gleichzeitigen Zusatz von pH-regulierender Lösung (4-molare NaOH) und Glucose-Lösung definierter Konzentration (40° Brix (= gelöste Trockensubstanz (Saccharose) in Gew.-%)) wird die Ausbeute an Biomasse bezogen auf Nährlösung wesentlich gesteigert. Die benutzte BETA-Nährlösung hat folgende Zusammensetzung:

Tabelle I

| Zusammensetzung der BETA-Nährlösung (Einsatzmengen für 1 Liter Nährlösung) | |
|---|---|
| peptonisierte Milch | 10,00 g |
| Hefeextrakt | 12,00 g |
| Dextrose | 20,00 g |
| Tween 80 | 1,00 g |
| Kaliumphosphat | 2,00 g |
| Natriumacetat | 5,00 g |
| Di-Ammoniumhydrogencitrat | 2,00 g |
| Magnesiumsulfat x 7 $H_2O$ | 0,20 g |
| Mangansulfat x 4 $H_2O$ | 0,05 g |

Die Mikroorganismen werden abgeerntet und mit einem speziellen Membrantrennungsverfahren gewonnen. Die so erhaltene Biomasse wird mit physiologischer Kochsalzlösung gereinigt und als Reinkultur Obstprodukten zugesetzt.

Zur Fermentation von Obstprodukten wird diese Biomasse z.B. Apfelsaft, Pfirsichnektar, Kirschnektar zugesetzt. Die Verfahrensbedingungen im einzelnen sind im Rahmen der vorstehend angegebenen Grenzen für jedes einzelne Produkt aufgrund sensorischer und wirtschaftlicher Belange festzulegen.

Es wurde gefunden, daß auch bestimmte andere Bakterienstämme für das vorliegende Verfahren geeignet sind. So eignet sich beispielsweise auch der Mikroorganismus Lactobacillus casei subspecies casei, wie er unter der Nr. DSM 3173 bei der Deutschen Sammlung für Mikroorganismen in Göttingen hinterlegt worden ist. Dieser Stamm wurde bei den Versuchen mit Beta 3 bezeichnet.

Es handelt sich dabei um grampositive unbewegliche Stäbchen; Größe 0,8 auf 2-3 $\mu$m; meist in Ketten vorliegend. Sie bilden keine Sporen, sind fakultativ anaerob; homofermentativ, obligat saccharoklastisch; das Endprodukt der Vergärung von Glucose ist ausschließlich Milchsäure. Fast ausschließlich wird L( + )-Milchsäure gebildet.

Das Bakterium ist Katalase- und Nitratreduktase-negativ. Ein Wachstum findet zwischen 15°C und 45°C statt. Gluconat wird verwertet. Säurebildung erfolgt aus Ribose, Mannit, Sorbit, Glucose, Maltose, Lactose, Saccharose, Cellobiose, Trehalose und Salicin. Keine Säure wird gebildet aus Arabinose, Xylose, Rhamnose, Melibiose und Raffinose.

Das Peptidoglycan der Zellwand enthält keine Diaminopimelinsäure.

Von wenigen aus einer Zahl von über 100 getesteten Arten ausgewählten Stämmen, die für das vorliegende Verfahren geeignet sind, zeigten die beiden hier beschriebenen Stämme ein sensorisch hervorragendes Ergebnis.

Einige charakteristische Verfahrensergebnisse sind in der als Anlage beigefügten Tabelle II zusammengestellt, die eine Auswahl bevorzugter Produkte enthält. Für die Versuche wurden beide genannten Bakterienstämme verwendet. Die Unterschiede zwischen den beiden Bakterienstämmen waren im wesentlichen sensorischer Art.

5

Die Tabelle zeigt, daß für alle genannten Fruchtsaftprodukte bei einer Fermentation unterhalb pH 3,7 die geforderten Merkmale, nämlich mindestens 95 % L(+)-Milchsäure, bezogen auf die Gesamtmilchsäure bei absoluten Mengen an L(+)-Milchsäure von mindestens 5 g/l erreichbar sind. Dabei sind die übrigen Verfahrensbedingungen in dem beanspruchten Rahmen an die speziellen Ausgangsprodukte anzupassen. Wie aus der Tabelle zu erkennen ist, erfordern bestimmte Säfte eine höhere Einsaat an Biomasse und eine um etwa 50 % längere Fermentationszeit.

Die ebenfalls als Anlage beigefügte Tabelle III gibt anhand von drei Produkten Aufschluß über die Veränderungen in den Säure- und Zuckergehalten, die bei der Fermentation eintreten.

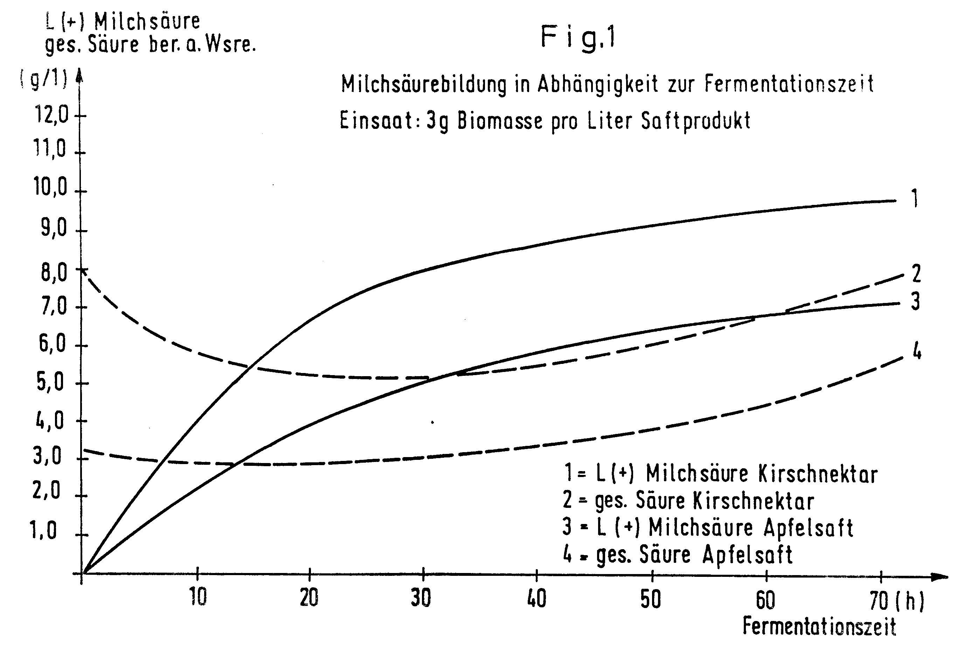

Über den Fermentationsverlauf bei einzelnen Fruchtsaftprodukten geben die als Anlage beigefügten Diagramme der Fig. 1-3 Aufschluß.

Das Diagramm der Fig. 1 zeigt den Gehalt an L(+)-Milchsäure und an Gesamtsäure, berechnet als Weinsäure in Abhängigkeit von der Fermentationszeit für Kirschnektar und Apfelsaft bei einer Einsaat von 3 g Biomasse pro Liter Saftprodukt. Danach wird der geforderte L(+)-Milchsäuregehalt für Apfelsaft erst nach einer Fermentationszeit von etwa 30 Stunden erreicht, während er sich bei Kirschnektar bereits nach etwa 13 Stunden einstellt. Das Diagramm zeigt ebenfalls, daß sehr lange Fermentationszeiten keine wesentliche Zunahme an L(+)-Milchsäure mehr zur Folge haben.

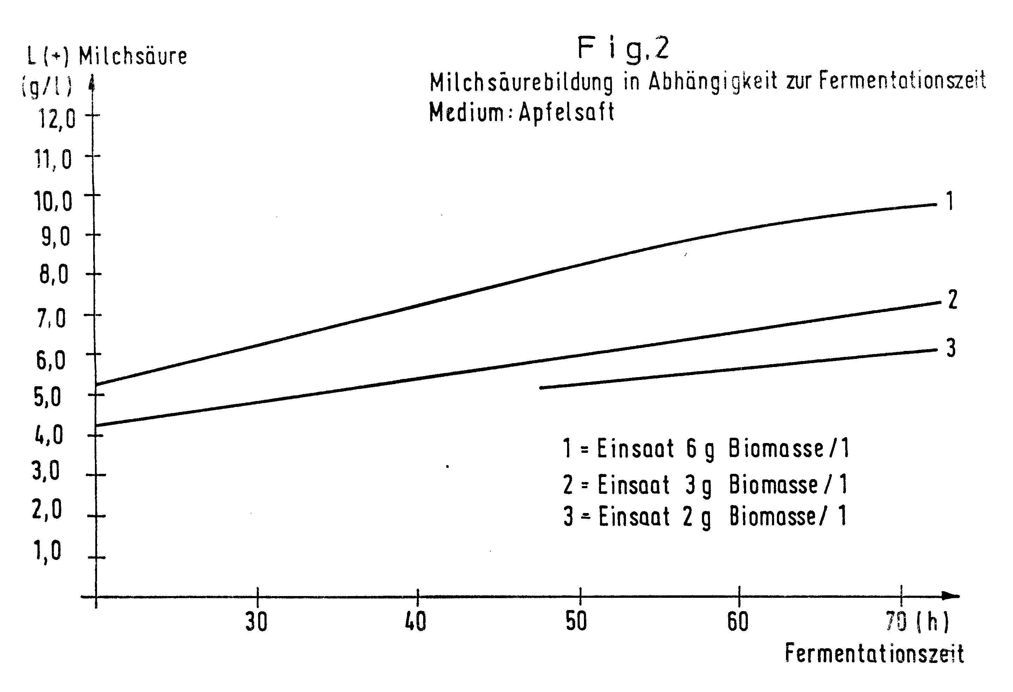

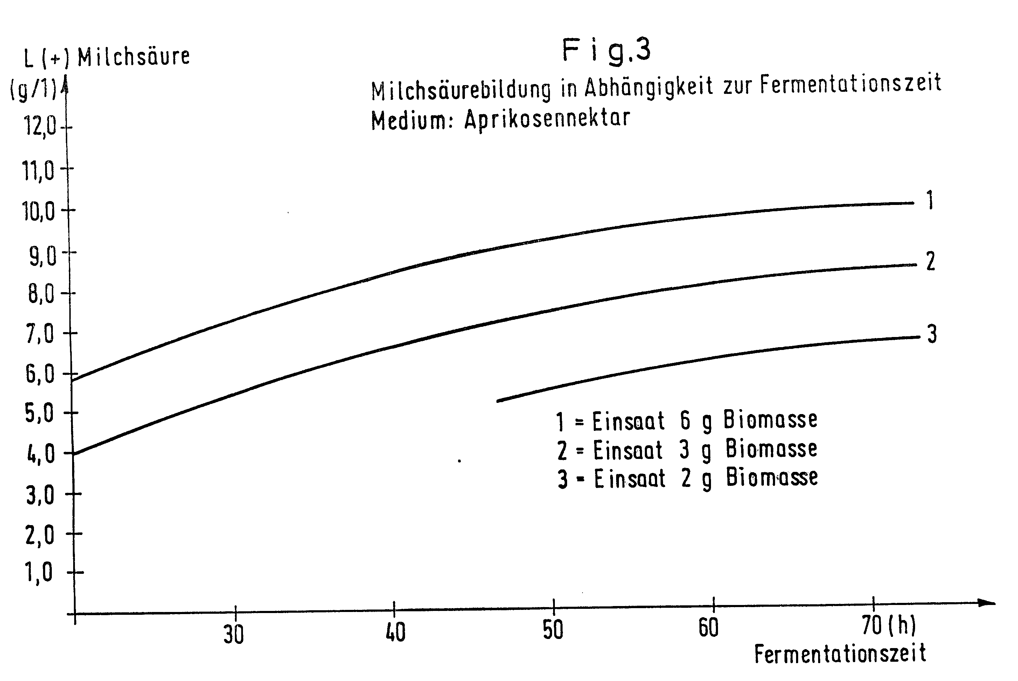

Die Diagramme der Fig. 2 und 3 zeigen die erreichbaren L(+)-Milchsäuregehalte einmal für Apfelsaft und zum anderen für Aprikosennektar bei unterschiedlichen Einsaatmengen an Biomasse.

Im folgenden sind noch vier Ausführungsbeispiele mit den Verfahrensbedingungen im einzelnen angegeben.

Ausführungsbeispiel 1

Lactofermentation von naturtrübem Apfelsaft

a) Anzucht der Biomasse

Die lyophilisierte Bakterienkultur wird mit physiologischer Kochsalzlösung aufgeschwemmt und in 50 ml MRS-Broth überführt. Nach 24 Stunden bei 33°C wird die gesamte Erstanzucht in 500 ml MRS-Broth 24 Stunden bei 33°C bebrütet. Dies ergibt etwa 7,5 g Biomasse. Die so erhaltene Biomasse wird unter sterilen Bedingungen abgeerntet und zur Fermenteranzucht verwendet. Im Fermenter wird BETA-Nährlösung steril vorgelegt. Die Beimpfung erfolgt mit 1 g der aus der zweiten Anzucht erhaltenen Biomasse pro Liter Broth. Durch gleichzeitigen Zusatz von pH-regulierender Lösung und Glucoselösung definierter Konzentration wird eine Ausbeute an Biomasse von ca. 55 g/l erreicht.

Nach beendeter Anzucht (24 Stunden bei 33°C) wird die Fermentationslösung im Sterilgefäß aufgenommen und unter sterilen Bedingungen die Biomasse aus der verbrauchten Nährlösung durch ein Membrantrennverfahren gewonnen. Die Lactobacillen werden in physiologischer Kochsalzlösung gereinigt und nochmals separiert. Die Biomasse liegt als Reinkultur vor und besteht aus einer weißen hochviskosen Masse.

b) Durchführung der Fermentation

100 l naturtrüber Apfelsaft mit einem pH-Wert von 3,4 wird über einen Plattenapparat (HKZE 90°C; HKZE = Hochkurzzeiterhitzung) im sterilen Tank eingelagert. Die Beimpfung erfolgt mit 300 g aus a) gewonnener Biomasse (entsprechend 3 g/l Saft). Bei großen Produktionsgebinden wird dabei ein langsames Rühren des Saftes erforderlich. Nach 72 Stunden Fermentationszeit bei 33°C haben sich ca. 7,5 g pro Liter L(+)Milchsäure gebildet. Die Milchsäure des so erzeugten Saftproduktes setzt sich aus etwa 99 Gew.% L-(+)-Milchsäure und 1 % D(-)-Milchsäure zusammen. Der milchsauer fermentierte, naturtrübe Apfelsaft wird mit 50 l nicht fermentierter Ausgangsware auf 5 g L(+)-Milchsäure pro Liter Endprodukt eingestellt und per Heißfüllung handelsüblich abgefüllt. Die Fermentation wurde sowohl mit Lactobacillus casei, subspecies casei (Stamm Beta 3) als auch mit Lactobacillus spec. (Stamm Beta 8) durchgeführt. Die Ergebnisse zeigten nur Unterschiede sensorischer Art.

Ausführungsbeispiel 2

Lactofermentation von Traubensaft

10 l roter bzw. weißer Traubensaft mit einem pH-Wert von 3,4 werden im sterilen Container über Plattenapparat (HKZE 85°C) eingelagert und mit 6 g Biomasse des Stammes Beta 8 pro Liter Saft beimpft. Die Bakterienanzucht erfolgt wie im Beispiel 1a) bereits beschrieben. Nach 72 Stunden bei 33°C haben sich 7,0 g/l L(+)-Milchsäure gebildet; der Anteil dieser ernährungsphysiologisch wichtigen L(+)-Milchsäure beträgt 96 %. Nach der Fermentation werden die Lactobacillen mittels Separator abgetrennt. Bei Ausführen dieses Verfahrensschrittes ist eine Wiederverwendung der Keime möglich. Das fermentierte Produkt wird mit 4 l Frischsaft anschließend auf 5,0 g/l L(+)-Milchsäure eingestellt, filtriert und weinsteinstabilisiert, dann HKZE bei 85°C auf Flaschen gefüllt.

Ausführungsbeispiel 3

Lactofermentation von Pfirsichnektar

10 l Pfirsichnektar (hergestellt aus Pfirsichmark und Zuckerwasser im Volumenverhältnis von 1:1) mit einem Zuckergehalt von 15° Brix werden über einen Plattenapparat (HKZE 110°C) steril im Fermentationstank eingelagert. Die Beimpfung erfolgt mit 30 g wie aus Beispiel 1 a) gewonnener Biomasse des Stammes Beta 8. Nach 48 Stunden bei 33°C sinkt der pH-Wert von 3,6 auf 3,4. Die L(+)-Milchsäurekonzentration liegt bei 6,1 g/l. Die Milchsäure besteht zu 95 % aus L(+)- und zu 5 % aus D(-)-Milchsäure.

Die fermentierte Ware wird mit 2,5 l nicht fermentierter Ausgangsware auf 5 g/l L(+)-Milchsäure verschnitten und nach HKZE (110°C) auf Flaschen gefüllt.

Ausführungsbeispiel 4

Herstellung von lactofermentierter Kirschkonfitüre

10 l Kirschmark von 15° Brix und mit einem pH-Wert von 3.4 werden einer Lactofermentation unterzogen, indem das Mark kurzzeitig bei 85°C pasteurisiert (HKZE 85°) und in einem sterilen Tank eingelagert wird. Dem Mark werden dann 6 g Biomasse des Stammes Beta 3 pro Liter beigefügt. Nach 72 Stunden Fermentationszeit bei 33°C haben sich 11 g/l L(+)-Milchsäure gebildet. Der Anteil an L(+)-Milchsäure beträgt 98 % der gebildeten Gesamtmilchsäure.

Das fermentierte Mark wird in einem Dünnschichtverdampfer 3-fach konzentriert und unter Zusatz von Zucker und Bindemittel zu einer gebrauchsfertigen Kirschkonfitüre gemischt und sterilisiert.

## Tabelle II

| Produkt | Biomasse-Einsaat (g/l) | pH-Wert vor Fermentation | pH-Wert nach Fermentation | Fermentationsbedingungen | | gebildete L(+)Milchsäure | |
|---|---|---|---|---|---|---|---|
| | | | | Zeit (h) | Temp. (°C) | L(+) Milchsäure (g/l) | Anteil (%) |
| Apfelsaft, naturtrüb | 3,0 | 3,4 | 3,4 | 72 | 33 | 7,8 | 96 |
| Apfelsaft | 3,0 | 3,6 | 3,4 | 72 | 33 | 7,5 | 99 |
| Traubensaft, weiß | 6,0 | 3,4 | 3,4 | 72 | 33 | 6,8 | 96 |
| Traubensaft, rot | 6,0 | 3,4 | 3,2 | 72 | 33 | 7,0 | 96 |
| Orangensaft | 6,0 | 3,6 | 3,2 | 72 | 20 | 7,2 | 95 |
| Pfirsichnektar | 3,0 | 3,5 | 3,2 | 48 | 33 | 5,7 | 95 |
| Aprikosennektar | 3,0 | 3,4 | 3,3 | 48 | 33 | 5,5 | 95 |
| Kirschnektar | 3,0 | 3,4 | 3,3 | 48 | 33 | 9,7 | 98 |
| Schw. Joh. Nektar | 6,0 | 3,3 | 3,1 | 72 | 33 | 5,2 | 96 |

EP 0 195 213 B1

## Tabelle III

### Analysendaten milchsauer fermentierter Frodukte

| | | Apfelsaft naturtrüb | | Pfirsichnektar | | Traubensaft, weiß | |
|---|---|---|---|---|---|---|---|
| | | O-Probe | ferm.Probe | O-Probe | ferm.Probe | O-Probe | ferm.Probe |
| Zuckergehalt | °Brix | 12,0 | 11,9 | 15,1 | 15,0 | 17,0 | 16,8 |
| pH-Wert | | 3,3 | 3,4 | 3,6 | 3,4 | 3,6 | 3,6 |
| ges. Säure*) | g/l | 7,3 | 4,6 | 3,1 | 6,0 | 7,9 | 7,1 |
| L(+)-Milchsäure | g/l | --- | 7,0 | --- | 6,1 | --- | 5,7 |
| D(-)-Milchsäure | g/l | --- | 0,1 | --- | 0,3 | --- | 0,1 |
| Äpfelsäure | g/l | 7,3 | n.n. | 1,8 | n.n. | 5,0 | 0,1 |
| Citronensäure | g/l | 0,2 | 0,1 | 1,8 | 1,6 | 0,4 | 0,3 |
| Glucose | g/l | 25,4 | 20,9 | 43,5 | 39,1 | 90,2 | 75,8 |
| Fructose | g/l | 64,8 | 58,3 | 43,3 | 38,8 | 89,5 | 76,9 |
| Saccharose | g/l | 22,0 | 20,0 | 44,0 | 44,0 | n.n. | n.n. |
| Einsaat Biomasse | g/l | --- | 3,0 | --- | 3,0 | --- | 3,0 |
| Fermentationszeit | h | --- | 72 | --- | 48 | --- | 66 |
| Fermentationstemp. | °C | --- | 33 | --- | 33 | --- | 33 |

n.n. = nicht nachweisbar

*) berechnet bei Apfelsaft als Weinsäure,
   bei Pfirsichnektar als Citronensäure,
   bei Traubensaft als Weinsäure

Patentansprüche

1. Verfahren zur Lactofermentation von Fruchtprodukten, bei dem das Ausgangsprodukt in Form von Maische oder Saft nach Abtöten der in ihm enthaltenen wilden Mikroflora einer Fermentation durch

EP 0 195 213 B1

milchsäureerzeugende Bakterien unterzogen wird, die als Fermentationsprodukt überwiegend L(+)-Milchsäure erzeugen, dadurch gekennzeichnet, daß Bakterien verwendet werden, deren milchsäureerzeugende Stoffwechselvorgänge auch noch bei pH-Werten kleiner 3,7 in Gang gebracht werden und ablaufen, und daß die Fermentation bei pH-Werten kleiner 3,7 durchgeführt und dabei ein Anteil an L-(+)-Milchsäure von mindestens 95 Gew.-% gebildet wird.

2. Verfahren nach Anspruch 1, dadurch gekennzeichnet, daß der pH-Wert am Ende der Fermentation kleiner als 3,5 ist.

3. Verfahren nach Anspruch 1 oder 2, dadurch gekennzeichnet, daß die Fermentation bis zu einem Gehalt von mindestens 5 g L(+)-Milchsäure je Liter Fermentationsprodukt durchgeführt wird.

4. Verfahren nach einem der Ansprüche 1 bis 3, dadurch gekennzeichnet, daß die Fermentation durch Zugabe von Biomasse in Form einer Reinzuchtkultur des milchsäureerzeugenden Bakteriums zum Ausgangsprodukt durchgeführt wird.

5. Verfahren nach Anspruch 4, dadurch gekennzeichnet, daß dem Ausgangsprodukt mindestens 1 g, vorzugsweise 2 - 6 g gereinigte, feuchte Reinzucht-Biomasse je Liter zugegeben wird.

6. Verfahren nach einem der Ansprüche 1 bis 5, dadurch gekennzeichnet, daß die Fermentationszeit mindestens 24 Stunden beträgt.

7. Verfahren nach Anspruch 6, dadurch gekennzeichnet, daß die Fermentationszeit bis zu 100 Stunden beträgt.

8. Verfahren nach einem der Ansprüche 1 bis 7, dadurch gekennzeichnet, daß die Fermentation bei Temperaturen zwischen 15 und 40°C durchgeführt wird.

9. Verfahren nach Anspruch 8, dadurch gekennzeichnet, daß die Fermentation bei Temperaturen zwischen 25°C und 35°C durchgeführt wird.

10. Verfahren nach einem der Ansprüche 4 bis 9, dadurch gekennzeichnet, daß die Biomasse in einem an sich bekannten, getrennten Vermehrungsverfahren erzeugt, von der Vermehrungsnährlösung abgetrennt und gereinigt wird.

11. Verfahren nach einem der Ansprüche 1-10, dadurch gekennzeichnet, daß zur Fermentation Lactobacillus spec., wie hinterlegt unter der Nr. DSM 3174, verwendet wird.

12. Verfahren nach einem der Ansprüche 1-10, dadurch gekennzeichnet, daß zur Fermentation Lactobacillus casei subspecies casei verwendet wird, wie hinterlegt unter der Nr. DSM 3173.

13. Verfahren nach einem der Ansprüche 1 bis 12, dadurch gekennzeichnet, daß das Fermentationsprodukt zu verkaufsfertigem Fruchtsaft aufbereitet wird.

14. Verfahren nach einem der Ansprüche 1 bis 12, dadurch gekennzeichnet, daß das Fermentationsprodukt zu Konfitüren, Gelees, Brotaufstrichen und Fruchtriegeln aufbereitet wird.

15. Verfahren nach einem der Ansprüche 1-14, dadurch gekennzeichnet, daß die Fermentation bis zu einem Äpfelsäuregehalt des Fermentationsproduktes, der gleich oder kleiner als 0,5 Gew.-% ist, geführt wird.

16. Verfahren nach einem der Ansprüche 1-14, dadurch gekennzeichnet, daß die Fermentation bis zu einem Äpfelsäuregehalt des Fermentationsproduktes, der gleich oder kleiner als 0,1 Gew.-% ist, geführt wird.

17. Lactobacillus spec., wie hinterlegt unter Nr. DSM 3174.

**18.** Verwendung von Lactobacillus spec., wie hinterlegt unter Nr. DSM 3174 zur milchsauren Fermentation von Fruchtsaftprodukten.

**19.** Fruchtprodukt, vorzugsweise Fruchtsaft, dadurch gekennzeichnet, daß es einen pH-Wert von kleiner 3.7, und einen Mindestgehalt an L(+)-Milchsäure von 5 g/l aufweist.

**20.** Fruchtprodukt nach Anspruch 19, dadurch gekennzeichnet, daß es einen Gehalt an Äpfelsäure von weniger als 1 g/l, vorzugsweise von weniger als 0,2 g/l aufweist.

**21.** Fruchtprodukt nach Anspruch 19 oder 20, dadurch gekennzeichnet, daß der pH-Wert kleiner 3,5 ist.

**Claims**

**1.** A process for the lactic fermentation of fruit products wherein, after the wild microflora contained in the initial product has been distroyed, the said initial product in the form of a fruit mash or fruit juice is subjected to a fermentation by lactic acid producing bacteria preponderantly producing L(+)-lactic acid as the fermentation product,
characterized in
that bacteria are used the lactic acid producing metabolic pathways of which can still be launched and proceed at a pH of less than 3.7, and in that the fermentation is performed at a pH of less than 3.7 in producing a share of L(+)-lactic acid of at least 95 per cent by weight.

**2.** A process according to claim 1, characterized in that, at the end of the fermentation, the pH is less than 3.5.

**3.** A process according to claims 1 or 2, characterized in that the fermentation is executed until a content of at least 5 g of the L(+)-lactic acid per liter of the fermentation product is formed.

**4.** A process according to anyone of claims 1 to 3, characterized in that the fermentation is carried out by adding a biomass in the form of a pure culture of the lactic acid producing bacteria to the initial product.

**5.** A process according to claim 4, characterized in that at least 1 g, preferably 2 to 6 g of a purified, moist pure-biomass per liter of the initial product is added to the initial product.

**6.** A process according to anyone of claims 1 to 5, characterized in that the fermentation time takes at least 24 hours.

**7.** A process according to claim 6, characterized in that the fermentation time takes up to 100 hours.

**8.** A process according to anyone of claims 1 to 7, characterized in that the fermentation is executed at temperatures of between 15 and 40° C.

**9.** A process according to claim 8, characterized in that the fermentation is executed at temperatures between 25° C and 35° C.

**10.** A process to anyone of claims 4 to 9, characterized in that the biomass is produced according to a generally known separate propagation process, thereafter separated from the nutrient growth solution, and then purified.

**11.** A process according to anyone of claims 1 to 10, characterized in that lactobacillus spec., as deposited under number DSM 3174, is used for the fermentation.

**12.** A process according to anyone of claims 1 to 10, characterized in that lactobacillus casei subspecies casei, as depostited under No. DSM 3137, is used for the fermentation.

**13.** A process according to anyone of claims 1 to 12, characterized in that the fermentation product is processed to a ready-to-sale fruit juice.

**14.** A process according to anyone of claims 1 to 12, characterized in that the fermentation product is processed to preserves, jelly, spread and fruit bars.

**15.** A process according to anyone of claims 1 to 14, characterized in that the fermentation is executed until the fermentation product has reached a content of malic acid being equal or less than 0.5 % by weight.

**16.** A process according to anyone of claims 1 to 14, characterized in that the fermentation is executed until the fermentation product has reached a content of malic acid being equal or less than 0.1 % by weight.

**17.** Lactobacillus spec. as deposited under No. DSM 3174.

**18.** The use of lactobacillus spec. as deposited under No. DSM 3174 for the lactic fermentation of fruit juice products.

**19.** A fruit product, preferably a fruit juice, characterized in that the said fruit product has a pH of less than 3.7 and a content of L(+)-lactic acid of at least 5 g/l.

**20.** Fruit product according to claim 19, characterized in that the said fruit product has a content of malic acid of less than 1 g/l, preferably of less than 0.2 g/l.

**21.** A fruit product according to claims 19 or 20, characterized in that the pH is less than 3.5.

**Revendications**

**1.** Procédé de fermentation lactique de produits à base de fruits, dans lequel le produit de départ sous la forme de bouillie ou de jus est, après destruction de la microflore sauvage contenue dedans, soumis à une fermentation par des bactéries génératrices d'acide lactique qui produisent, comme produit de fermentation, principalement de l'acide L(+)-lactique, caractérisé en ce qu'on utilise des bactéries dont les processus métaboliques de production d'acide lactique sont mis en marche et se déroulent également encore à des valeurs de pH inférieures à 3,7 et en ce que la fermentation est effectuée à des valeurs de pH inférieures à 3,7, une fraction d'acide L(+)-lactique d'au moins 95 % en poids étant alors formée.

**2.** Procédé suivant la revendication 1, caractérisé en ce que la valeur du pH à la fin de la fermentation est inférieure à 3,5.

**3.** Procédé suivant l'une des revendications 1 et 2, caractérisé en ce que la fermentation est effectuée jusqu'à une teneur d'au moins 5 g d'acide L(+)-lactique par litre de produit de fermentation.

**4.** Procédé suivant l'une des revendications 1 à 3, caractérisé en ce que la fermentation est effectuée par addition au produit de départ d'une biomasse sous la forme d'une culture pure de la bactérie produisant de l'acide lactique.

**5.** Procédé suivant la revendication 4, caractérisé en ce qu'au produit de départ on ajoute par litre au moins 1 g, de préférence 2-6 g, de biomasse de culture pure humide, purifiée.

**6.** Procédé suivant l'une des revendications 1 à 5, caractérisé en ce que le temps de fermentation est d'au moins 24 heures.

**7.** Procédé suivant la revendication 6, caractérisé en ce que le temps de fermentation est de jusqu'à 100 heures.

**8.** Procédé suivant l'une des revendications 1 à 7, caractérisé en ce que la fermentation est effectuée à des températures comprises entre 15 et 40°C.

**9.** Procédé suivant la revendication 8, caractérisé en ce que la fermentation est effectuée à des températures comprises entre 25°C et 35°C.

**10.** Procédé suivant l'une des revendications 4 à 9, caractérisé en ce que la biomasse est produite dans un procédé de multiplication séparé, connu en soi, est isolée de la solution nutritive de multiplication et est purifiée.

**11.** Procédé suivant l'une des revendications 1 à 10, caractérisé en ce que, pour la fermentation, on utilise Lactobacillus spec., tel que déposé sous le n° DSM 3174.

**12.** Procédé suivant l'une des revendications 1 à 10, caractérisé en ce que, pour la fermentation, on utilise Lactobacillus casei subspecies casei, tel que déposé sous le n° DSM 3173.

**13.** Procédé suivant l'une des revendications 1 à 12, caractérisé en ce que le produit de fermentation est traité en un jus de fruit prêt à la vente.

**14.** Procédé suivant l'une des revendications 1 à 12, caractérisé en ce que le produit de fermentation est traité en confitures, gelées, pâtes à tartiner et pâtes de fruits.

**15.** Procédé suivant l'une des revendications 1 à 14, caractérisé en ce que la fermentation est conduite jusqu'à une teneur en acide malique du produit de fermentation qui soit égale ou inférieure à 0,5 % en poids.

**16.** Procédé suivant l'une des revendications 1 à 14, caractérisé en ce que la fermentation est conduite jusqu'à une teneur en acide malique du produit de fermentation qui soit égale ou inférieure à 0,1 % en poids.

**17.** Lactobacillus spec, tel que déposé sous le n° DSM 3174.

**18.** Utilisation de Lactobacillus spec., tel que déposé sous le n° DSM 3174, pour la fermentation lactique de produits de jus de fruits.

**19.** Produit à base de fruits, de préférence jus de fruits, caractérisé en ce qu'il présente une valeur de pH inférieure à 3,7 et une teneur minimale en acide L(+)-lactique de 5 g/litre.

**20.** Produit à base de fruits suivant la revendication 19, caractérisé en ce qu'il présente une teneur en acide malique inférieure à 1 g/litre, de préférence inférieure à 0,2 g/litre.

**21.** Produit à base de fruits suivent l'une des revendications 19 et 20, caractérisé en ce que la valeur du pH est inférieure à 3,5.

# Fig.1

Milchsäurebildung in Abhängigkeit zur Fermentationszeit

Einsaat: 3g Biomasse pro Liter Saftprodukt

L (+) Milchsäure
ges. Säure ber. a. Wsre.

(g/l)
12,0
11,0
10,0
9,0
8,0
7,0
6,0
5,0
4,0
3,0
2,0
1,0

10   20   30   40   50   60   70 (h)

Fermentationszeit

1 = L (+) Milchsäure Kirschnektar
2 = ges. Säure Kirschnektar
3 = L (+) Milchsäure Apfelsaft
4 = ges. Säure Apfelsaft

14

F i g.2

Milchsäurebildung in Abhängigkeit zur Fermentationszeit
Medium: Apfelsaft

L (+) Milchsäure
(g/l)

1 = Einsaat 6 g Biomasse/1
2 = Einsaat 3 g Biomasse/1
3 = Einsaat 2 g Biomasse/1

Fermentationszeit

EP 0 195 213 B1

F i g. 3

Milchsäurebildung in Abhängigkeit zur Fermentationszeit
Medium: Aprikosennektar

L (+) Milchsäure
(g/l)

12,0
11,0
10,0
9,0
8,0
7,0
6,0
5,0
4,0
3,0
2,0
1,0

1
2
3

1 = Einsaat 6 g Biomasse
2 = Einsaat 3 g Biomasse
3 = Einsaat 2 g Biomasse

30   40   50   60   70 (h)

Fermentationszeit

16